(19) Europäisches Patentamt
European Patent Office
Office européen des brevets

(11) Veröffentlichungsnummer: **0 115 810**
**B1**

(12) # EUROPÄISCHE PATENTSCHRIFT

(45) Veröffentlichungstag der Patentschrift: **21.08.85**

(51) Int. Cl.⁴: **C 07 C 51/567**, C 07 C 63/70

(21) Anmeldenummer: **84100688.5**

(22) Anmeldetag: **24.01.84**

(54) **Verfahren zur Herstellung von Fluorphthalsäureanhydriden.**

(30) Priorität: **02.02.83 DE 3303378**

(43) Veröffentlichungstag der Anmeldung:
**15.08.84 Patentblatt 84/33**

(45) Bekanntmachung des Hinweises auf die Patenterteilung:
**21.08.85 Patentblatt 85/34**

(84) Benannte Vertragsstaaten:
**CH DE FR GB IT LI**

(56) Entgegenhaltungen:
**EP - A - 0 003 344**
**DE - A - 1 468 395**

**BULLETIN OF THE CHEMICAL SOCIETY OF JAPAN, Band 48, Nr. 1, Januar 1975, Seiten 359-360 N. ISHIKAWA et al.: "The preparation of 3- and 4-fluorophtalic anhydrides by fluorodenitration"**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Varwig, Juergen, Dr., Bitterstrasse 21, D-6900 Heidelberg (DE)**
Erfinder: **Rohr, Wolfgang, Dr., In der Dreispitz 13, D-6706 Wachenheim (DE)**

**EP 0 115 810 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von Fluorphthalsäureanhydriden durch Behandlung der entsprechenden Chlorphthalsäureanhydride in Gegenwart eines aliphatischen Sulfons mit einem Säurechlorid der schwefeligen Säure oder der Kohlensäure und nachfolgende Umsetzung mit Kaliumfluorid.

Es ist aus J. Org. Chem. 25, 834 (1960) bekannt, daß man 3-Fluorphthalsäureanhydrid durch Erhitzen von 3-Chlorphthalsäureanhydrid mit überschüssigem Kaliumfluorid herstellen kann. Bei diesem Verfahren wird 3-Fluorphthalsäureanhydrid in einer Ausbeute von 50% erhalten. Das gleiche Verfahren wird auch in der EP-A-55 630 beschrieben, in der angegeben ist, daß nach mehrstündigem Eritzen auf 235° C ein Reaktionsgemisch erhalten wird, aus dem man durch Destillation und Umkristallisation ein in seiner Reinheit nicht charakterisiertes 3-Fluorphthalsäureanhydrids mit einer Ausbeute von 68% erhält. Bei der entsprechenden Herstellung des 4,5-Difluorphthalsäureanhydrids, beträgt die Ausbeute nur 66%. Man kann diese Umsetzung nach den Angaben auf Seite 4, Zeilen 29—33 der EP-A-55 630 auch in Gegenwart dipolarer aprotischer Lösungsmittel, wie Sulfolan durchführen, doch ist die lösungsmittelfreie Mischung bevorzugt.

Nach Beispiel 8 der US-PS 2 891 074 erhält man durch 24stündiges Erhitzen von 4-Chlorphthalsäureanhydrid mit einem großen Überschuß an Kaliumfluorid in einem Autoklaven auf 200° C 4-Fluorphthalsäureanhydrid in einer Ausbeute von 52%.

Es wurde nun gefunden, daß man Fluorphthalsäureanhydride der Formel

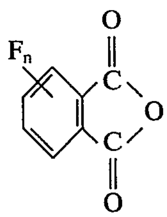

(I)

in der n 1 oder 2 bedeutet, durch Umsetzung von Chlorphthalsäureanhydriden der Formel

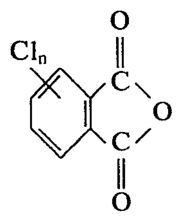

(II)

in der n die vorgenannte Bedeutung besitzt, mit Kaliumfluorid in Gegenwart eines aliphatischen Sulfons als Lösungsmittel erheblich vorteilhafter herstellen kann, wenn man das Chlorphthalsäureanhydrid in Gegenwart des aliphatischen Sulfons bei Temperaturen bis 150° C mit einem Säurechlorid der schwefeligen Säure oder Kohlensäure behandelt und das Reaktionsgemisch dann bei Temperaturen von 150 bis 250° C mit Kaliumfluorid umsetzt.

Im Vergleich zu den bekannten Verfahren liefert das Verfahren nach der Erfindung ein erheblich verbessertes Ergebnis in bezug auf Ausbeute und Reinheit der Fluorphthalsäureanhydride. Wesentlich übertroffen werden die bekannten Herstellungsverfahren auch durch kürzere Reaktionszeiten und einen geringeren Bedarf an Kaliumfluorid.

Nach dem neuen Verfahren werden die Chlorphthalsäureanhydride der Formel II, das sind beispielsweise 3-Chlorphthalsäureanhydrid, 4-Chlorphthalsäureanhydrid, 3,6-N,N-Dichlorphthalsäureanhydrid, 4,5-Dichlorphthalsäureanhydrid, 3,5-Dichlorphthalsäureanhydrid und 3,4-Dichlorphthalsäureanhydrid, in einer ersten Stufe des Verfahrens in Gegenwart eines aliphatischen Sulfons bei Temperaturen bis 150° C mit einem Säurechlorid der schwefligen Säure oder Kohlensäure, wie Thionylchlorid oder Phosgen, behandelt.

Als aliphatische Sulfone sind z. B. die Verbindungen der Formel

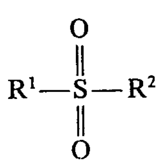

(III)

geeignet, in der $R^1$ und $R^2$ gleich oder verschieden sind und jeweils einen aliphatischen Rest, vorzugsweise einen Alkylrest mit 1 bis 8, insbesondere 1 bis 4 Kohlenstoffatomen oder $R^1$ und $R^2$ zusammen einen Alkylenrest mit 4 oder 5 Kohlenstoffatomen bedeuten. Geeignete Lösungsmittel der genannten

Art sind beispielsweise Dimethylsulfon, Diethylsulfon, Dipropylsulfon, Diisopropylsulfon, Dibutylsulfon, Diisobutylsulfon, Methylethylsulfon, Tetramethylensulfon (= Sulfolan) und Pentamethylensulfon, von denen Sulfolan bevorzugt ist. Zweckmäßig verwendet man das Lösungsmittel in einer Menge von 50 bis 1000 Gewichtsprozent, vorzugsweise 100 bis 300 Gewichtsprozent, bezogen auf Ausgangsstoff II. Die Säurechloride werden zweckmäßig in einer Menge von 1 bis 20 Gewichtsprozent, vorzugsweise von 2 bis 12 Gewichtsprozent, bezogen auf das Sulfon III eingesetzt.

Die erste Verfahrensstufe wird bei einer Temperatur bis 150° C, zweckmäßig zwischen 50 und 120° C, insbesondere bei 70 bis 100° C durchgeführt. Dabei können der Ausgangsstoff II, das Sulfon III und das Säurechlorid in beliebiger Reihenfolge in dem angegebenen Temperaturbereich miteinander vermischt werden. Zweckmäßig vermischt man jedoch zunächst bei 30 bis 40° C den Ausgangsstoff II mit dem Sulfon III und gegebenenfalls mit einem Katalysator, fügt dann unter Rühren das Säurechlordid hinzu und erhitzt das Gemisch in dem angegebenen Temperaturbereich. Man erhitzt zweckmäßig so lange, bis keine Gasentwicklung mehr beobachtet wird. Es empfiehlt sich, überschüssige Säurechlorid z. B. durch Einblasen eines Inertgases, wie Stickstoff, oder durch Anlagen von Vakuum zu entfernen.

Als Katalysatoren sind für diese Verfahrensstufe z. B. N,N-disubstituierte Carbonamide mit 3 bis 10 Kohlenstoffatomen geeignet, wie N,N-Dimethylformamid, N,N-Dimethylacetamid, N,N-Diethylformamid, N,N-Di-n-propylacetamid, N-Methyl-N-ethyl-formamid oder N,N-Di-isopropylacetamid. Der Katalysator wird zweckmäßig in einer Menge von 0,2 bis 2 Gewichtsprozent bezogen auf das Säurechlorid eingesetzt.

Das Reaktionsgemisch aus der ersten Verfahrensstufe wird in einer zweiten Stufe bei Temperaturen von 150 bis 250°C insbesondere von 170 bis 240°C, bevorzugt von 190 bis 220°C mit Kaliumfluorid umgesetzt. Man kann dabei drucklos oder unter Druck, kontinuierlich oder diskontinuierlich arbeiten. Das Kaliumfluorid wird z. B. in einer Menge von 1 bis 1,4, vorzugsweise 1,1 bis 1,2 Mol, pro Grammäquivalent Chlor in dem Ausgangsstoff II angewendet. Man verfährt z. B. folgendermaßen: Zu dem Gemisch, das man aus der ersten Verfahrensstufe erhält, gibt man das Kaliumfluorid, das zweckmäßig vorher getrocknet wurde und hält die durch Verrühren erhaltene Mischung 1 bis 10 Stunden auf Reaktionstemperatur.

Der Halogenaustausch verläuft bereits ohne Katalysator mit hoher Geschwindigkeit. Er kann jedoch durch Zugabe eines Kronenethers oder Cryptanden beschleunigt werden. Diese sind organische Komplexliganden, die insbesondere gut zur Bildung von Alkali dienen. Kronenether sind cyclisch angeordnete, neutrale Ethylenglykolether. Die Cryptanden liefern eine dreidimensionale Umhüllung. Bezüglich Herstellung dieser Stoffe, siehe »Kontakte« (1977), Seiten 11 bis 31 und 36 bis 48. Als Katalysator für die zweite Stufe des erfindungsgemäßen Verfahrens sind Kronenether bevorzugt, von denen z. B. die folgenden Verbindungen genannt sein sollen: 12-Krone-4; 2,4,6,8-Methyl-12-krone-4; 14-Krone-4; Dibenzo-14-krone-4; Dibutyl-benzo-14-krone-4; Dicyclohexyl-14-krone-4; 15-Krone-5; 1,2-Benzo-15-krone-5; 1,2-Butylbenzo—15-krone-5; 1,2-Cyclohexyl-15-krone-5; Dibenzo-15-krone-5; 16-Krone-5; Dibenzo-16-krone-5; 18-Krone-5; Dibenzo-18-krone-5; 18-Krone-6; Benzo-18-krone-6; Cyclohexyl-18-krone-6; Dibenzo-18-krone-6; Dicyclohexyl-18-krone-6; Tribenzo-18-krone-6; Dinaphtho-18-krone-6; 19-Krone-6; Dibenzo-19-krone-6; 20-Krone-7; Dibenzo-5-oxy-20-krone-7; 21-Krone-7; Dibenzo-21-krone-7; Dicyclohexyl-21-krone-7; 24-Krone-8; Dibenzo-24-krone-8; Dicyclohexyl-24-krone-8; Tetrabenzo-24-krone-8; 30-Krone-10; 40-Krone-20; Aza-18-krone-6; Dibenzo-aza-18-krone-6; Diaza-18-krone-6; Dibenzo-diaza-18-krone-6; 1,4-Dithia-15-krone-5; 1,4-Dithia-18-krone-6; 1,7-Dithia-benzo-18-krone-6; 1,10-Dithia-benzo-18-krone-6 und 1,7,10,16-Tetrathia-18-krone-6.

Diese Katalysatoren werden zweckmäßig in einer Menge von 0,05 bis 0,5, insbesondere 0,1 bis 0,3 Molprozent je Mol Ausgangsstoff II eingesetzt.

Nach der Umsetzung mit Kaliumfluorid, die etwa nach $^1/_2$ bis 8 Stunden beendet ist, arbeitet man auf an sich übliche Weise, z. B. durch Filtration, Waschen des Festgutes und Destillation von Filtrat und Waschfiltraten, auf. Da die nach dem erfindungsgemäßen Verfahren herstellbaren Fluorphthalsäureanhydride in einem ähnlichen Bereich wie Sulfolan destillieren, kann man durch Zugabe von etwa Sulfolan zu dem Destillationsrückstand noch vorhandene Reste an Endstoff I azeotrop extrahieren und nach dem Abkühlen des Destillats absaugen.

Die so erhaltenen Fluorphthalsäureanhydridderivate sind wertvolle Ausgangsstoffe für die Herstellung von Farbstoffen, Pharmazeutika und Pflanzenschutzmitteln.

Die in den flgenden Beispielen aufgeführten Teile bedeuten Gewichtsteile.

## Beispiel 1

Zu einer Mischung von 500 Teilen 3-Chlorphthalsäureanhydrid und 800 Teilen Sulfonan werden bei 40° C unter Rühren 96 Teile Thionylchlorid gegeben. Das Reaktionsgemisch wird innerhalb 20 Minuten auf 100° C erwärmt und 30 Minuten bei dieser Temperatur bis zum Ende der Gasabspaltung gerührt. Anschließend wird innerhalb von 10 Minuten überschüssiges Thionylchlorid im Wasserstrahlvakuum abgezogen, wobei die Temperatur auf 90° C absinkt. Bei gleicher Temperatur werden nun 190,9 Teile Kaliumfluorid in die Reaktionslösung eingetragen. Man rührt das Gemisch 3 Stunden bei 205° C. Durch Gaschromatographie einer Probe kann man neben Sulfolan völlig umgesetztes reines 3-Fluorphthalsäureanhydrid feststellen. Innerhalb der Nachweisgrenze ist 3-Chlorphthalsäureanhydrid nicht mehr nachweisbar.

Nach dem Abkühlen auf 40°C wird das Reaktionsgemisch abgesaugt und mit 100 Teilen Aceton nachgewaschen. Das Filtrat wird am Rotationsverdampfer bei 50°C/$_{10}$ mbar von Aceton befreit und dann über einen Topftrichter bei 200°C/$_{0,3}$ mbar in eine 500 Volumenteile-Destillationsapparatur so eingeführt, daß Sulfolan kontinuierlich mit 3-Fluorphthalsäureanhydrid über destilliert. Zur extraktiven Destillation verbleibender Restmengen an Endprodukt im Rückstand werden zum Schluß noch 126 g Sulfolan durch den Tropftrichter in die Destillationsapparatur eingeführt. Man erhält 1317,4 Teile Gesamtdestillat. Nach Abzug des Sulfolans (926 Teile) ergibt sich eine Ausbeute von 391,4 Teilen (86,1% d. Th.) 3-Fluorphthalsäureanhydrid. Es kristallisiert beim Erkalten des Destillats zu etwa einem Drittel aus. Durch Absaugen, Waschen mit Toluol und Pentan erhält man 117 Teile 3-Fluorphthalsäureanhydrid mit einem Schmelzpunkt von 158—160°C. Der Rest verbleibt im Filtrat gelöst. Nach der gaschromatographischen Untersuchung an einer 2 m SE 30-Säule (Chromosorb W, 80—100 mesh, belegt mit 20% SE 30 Silicon-Gummi) bei 220°C enthalten Rückstand und Filtrat das gleiche reine 3-Fluorphthalsäureanhydrid.

Beispiel 2

Zu einer Mischung von 240 Teilen 3-Chlorphthalsäureanhydrid und 504 Teilen Sulfolan werden bei 36°C unter Rühren 35 Teile Thionylchlorid gegeben. Das Reaktionsgemisch wird innerhalb 15 Minuten auf 110°C erwärmt. Dann rührt man bis zum Ende der Gasabspaltung weitere 25 Minuten bei gleicher Temperatur. Anschließend wird überschüssiges Thionylchlorid im Wasserstrahlvakuum innerhalb von 10 Minuten abgezogen.

In das Gemisch gibt man nun bei 90°C 84 Teile Kaliumfluorid und 0,7 Teile 18-Krone-6 und rührt 2 Stunden bei 210°C. Man kühlt ab und arbeitet wie in Beispiel 1 angegeben auf. Durch azeotrope Nachdestillation mit 126 Teilen Sulfolan werden 820 Teile Gesamtdestillat erhalten. Es enthält 190 Teile (87% d. Th.) 3-Fluorphthalsäureanhydrid. Eine abgesaugte und mit Roluol und Pentan gewaschene Probe weist einen Schmelzpunkt von 158 bis 160°C auf. Nach der gaschromatographischen Untersuchung enthält das Filtrat außer Sulfolan nur reines 3-Fluorphthalsäureanhydrid.

Beispiel 3

Zu einer Mischung von 50 Teilen 3,6-Dichlorphthalsäureanhydrid und 150 Teilen Sulfolan werden bei 35°C unter Rühren 16 Teile Tionylchlorid zugegeben. Das Reaktionsgemisch wird innerhalb 10 Minuten auf 105°C erwärmt und 20 Minuten bei gleicher Temperatur nachgerührt. Anschließend wird überschüssiges Thionylchlorid im Wasserstrahlvakuum während 10 Minuten abgezogen. In das heiße Gemisch gibt man nun 30,7 Teile Kaliumfluorid und rührt 2 Stunden bei 220°C. Man kühlt ab und arbeitet wie in Beispiel 1 angegeben, jedoch bei 225°C/0,3 mbar auf. Durch azeotrope Nachdestillation mit 30 Teilen Sulfolan werden 213,5 Teile Gesamtdestillat erhalten. Es enthält 33,5 Teile (79% d. Th.) 3,6-Difluorphthalsäureanhydrid. Eine abgesaugte und mit Toluol gewaschene Probe schmilzt zwischen 205 und 207°C. Nach der gaschromatographischen Untersuchung enthält das Filtrat außer Sulfolan nur reines 3,6-Difluorphthalsäureanhydrid.

Beispiel 4

50 Teile Phosgen werden während 40 Minuten bei 100°C unter Rühren in eine Mischung von 450 Teilen Sulfolan und 250 Teilen 4-Chlorphthalsäureanhydrid bei 100°C eingeleitet. Anschließend werden Phosgenreste im Wasserstrahlvakuum während 10 Minuten bei 100°C abgezogen. In das heiße Gemisch werden nun 95,4 Teile Kaliumfluorid eingetragen und 3$^1/_2$ Stunden bei 215°C gerührt. Nach dem Abkühlen, Aufarbeiten gemäß Beispiel 1 und azeotroper Nachdestillation mit 126 Teilen Sulfolan erhält man 753,2 Teile Gesamtdestillat. Es enthält 177,2 Teile (78% d. Th.) gaschromatographisch reines 4-Fluorphthalsäureanhydrid. Eine mit Wasser ausgefällte Probe weist einen Fp. von 78 bis 81°C auf.

**Patentansprüche**

1. Verfahren zur Herstellung von Fluorphthalsäureanhydriden der Formel

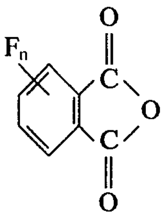

(I)

in der n 1 oder 2 bedeutet, durch Umsetzung der entsprechenden Chlorphthalsäureanhydride der Formel

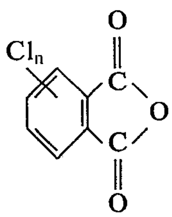

(II)

in der n die vorgenannte Bedeutung hat, mit Kaliumfluorid in Gegenwart eines aliphatischen Sulfons als Lösungsmittel, dadurch gekennzeichnet, daß man das Chlorphthalsäureanhydrid in Gegenwart des aliphatischen Sulfons bei Temperaturen bis 150° C mit einem Säurechlorid der schwefeligen Säure oder Kohlensäure behandelt und das Reaktionsgemisch dann bei Temperaturen von 150 bis 250° C mit Kaliumfluorid umsetzt.

2. Verfahren nach Anspruch 1, dadurch gekennzeichnet, daß man als Säurechlorid der schwefeligen Säure oder Kohlensäure Thionylchlorid oder Phosgen verwendet.

**Claims**

1. A process for the preparation of a fluorophthalic anhydride of the formula

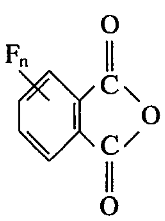

(I)

where n is 1 or 2, by reacting the corresponding chlorophthalic anhydride of the formula

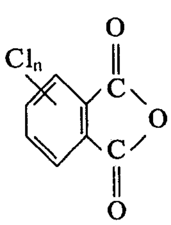

(II)

where n has the above meanings, with potassium fluoride in the presence of an aliphatic sulfone as a solvent, wherein the chlorophthalic anhydride is treated with an acid chloride of sulfurous acid or of carbonic acid in the presence of the aliphatic sulfone at up to 150° C, and the reaction mixture is then reacted with potassium fluoride at from 150 to 250° C.

2. A process as claimed in claim 1, wherein thionyl chloride or phosgene is used as the acid chloride of sulfurous acid or of carbonic acid.

**Revendications**

1. Procédé pour la préparation d'anhydrides d'acide fluorophthalique de formule

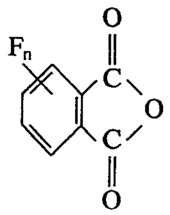

(I)

dans laquelle n est égal à 1 ou 2, par réaction de l'anhydride d'acide chlorophtalique correspondant de formule

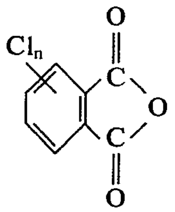

(II)

dans laquelle n a la signification donnée ci-dessus, avec le fluorure de potassium en présence d'une sulfone aliphatique servant de solvant, caractérisé en ce que l'on traite l'anhydride d'acide chlorophtalique par un chlorure de l'acide sulfureux ou de l'acide carbonique, en présence de la sulfone aliphatique et à des températures allant jusqu'à 150° C, et on fait réagir ensuite le mélange réactionnel avec le fluorure de potassium à des températures de 150 à 250° C.

2. Procédé selon la revendication 1, caractérisé en ce que l'on utilise le chlorure de thionyle ou le phosgène comme chlorure de l'acide sulfureux ou de l'acide carbonique.